Europäisches Patentamt

European Patent Office

Office européen des brevets

(11) Publication number: **0 387 861**
**A2**

# EUROPEAN PATENT APPLICATION

(21) Application number: **90104854.6**

(22) Date of filing: **14.03.90**

(51) Int. Cl.⁵: **C12N 15/31, C12P 21/02, C07K 13/00, //(C12P21/02, C12R1:645)**

(30) Priority: **14.03.89 JP 61280/89**

(43) Date of publication of application:
**19.09.90 Bulletin 90/38**

(84) Designated Contracting States:
**AT BE CH DE DK ES FR GB GR IT LI LU NL SE**

(71) Applicant: **NICHIREI CORPORATION**
**3-3-23 Misaki-cho Chiyoda-ku**
**Tokyo(JP)**

(72) Inventor: **Kobata, Akira**
**6-6-4-203, Ochiai**
**Tama-shi, Tokyo(JP)**
Inventor: **Kochibe, Naohisa**
**1168-32 Aramaki-cho**
**Maebashi-shi, Gunma(JP)**
Inventor: **Fukumori, Fumiyasu, c/o Laboratory**
**of**
**Nichirei Corporation, 52-14 Kumegawa-cho**
**1-chome**
**Higashimurayama-shi, Tokyo(JP)**
Inventor: **Takeuchi, Naomi, c/o Laboratory of**
**Nichirei Corporation, 52-14 Kumegawa-cho**
**1-chome**
**Higashimurayama-shi, Tokyo(JP)**

(74) Representative: **Kinzebach, Werner, Dr. et al**
**Patentanwälte Reitstötter, Kinzebach und**
**Partner Sternwartstrasse 4 Postfach 86 06 49**
**D-8000 München 86(DE)**

(54) **Cloned cDNA encoding lectin of mushroom aleuria aurantia and recombinant vector containing the same.**

(57) A cloned cDNA comprising a region encoding Aleuria aurantia lectin (AAL) and a recombinant vector containing the cDNA, which can express the cDNA to produce AAL are disclosed. The recombinant vector can be used for the production of Aleuria aurantia lectin in a large amount by genetic engineering technique.

# Cloned cDNA Encoding Lectin of Mushroom Aleuria aurantia and Recombinant Vector Containing the Same

## BACKGROUND OF THE INVENTION

### I. Field of the Invention

This invention relates to a cDNA encoding a lectin of mushroom Aleuria aurantia and an expression vector containing the cDNA for the production of the Aleuria aurantia lectin (hereinafter also referred to as "AAL" for short). The AAL has a use as a diagnostic for cancer and as a reagent for separating or analyzing various sugar chains.

### II. Description of the Related Art

Lectins are sugar-binding proteins and of great value as specific probes for investigating the structure and function of carbohydrate chains on the surface of animal cells. AAL has been purified from fruit bodies of orange peel mushroom, Aleuria aurantia (N. Kochibe and K. Furukawa, Biochemistry 19, 2841-2846 (1980)). Since AAL exhibits a unique binding property for fucosyl linkages (Yamashita et al., J. Biol. Chem. 260, 4688-4693, 1985). AAL recognizes the change of oligosaccharide chains in cell surface glycoproteins, so that it can be used as a diagnostic for diseases such as cancer in which the oligosaccharide chains are modified (Japanese Patent Application No. 233314/88, not yet laid open).

AAL is now prepared by extraction from the fruit bodies of Aleuria aurantia and subsequent purification of the extract. Although the mushroom Aleuria aurantia grows naturally in the mountains in Japan, since its fruit body can be harvested only in late summer to early autumn, it is difficult to stably produce AAL from the fruit body. Thus, it is desired to produce AAL by a genetic engineering technique.

## SUMMARY OF THE INVENTION

Accordingly, the object of the present invention is to provide means for producing AAL by a genetic engineering technique.

The present invention provides a cloned cDNA containing a region encoding Aleuria aurantia lectin.

The present invention also provides a recombinant vector containing the cDNA of the present invention, which can express the cDNA to produce AAL.

By the present invention, it was first accomplished to produce AAL by transforming a host such as E. coli with the recombinant vector of the present invention and recovering the AAL produced by the host. Thus, the present invention largely contributes to the stable and continuous production of AAL and, in turn, contributes to the development of diagnostics of cancers and to the analysis procedures of sugar chains.

## BRIEF DESCRIPTION OF THE DRAWINGS

Fig. 1 shows the nucleotide sequence of Aleuria aurantia lectin cDNA and its deduced amino acid sequence;

Fig. 2 shows the amino acid sequence of the amino-terminal region of natural AAL;

Fig. 3A shows the strategy for the construction of an AAL expression vector pKA-I;

Fig. 3B shows sequences of chemically synthesized polynucleotides used to construct pKA-I;

Fig. 4 shows the results of SDS-polyacrylamide gel electrophoresis analysis in the purification process of AAL from E. coli; and

Fig. 5 shows chromatograms of asparagine-linked oligosaccharides and human milk oligosaccharides on AAL-Sepharose column, the AAL being produced by the genetic engineering technique.

## DETAILED DESCRIPTION OF THE PREFERRED EMBODIMENTS

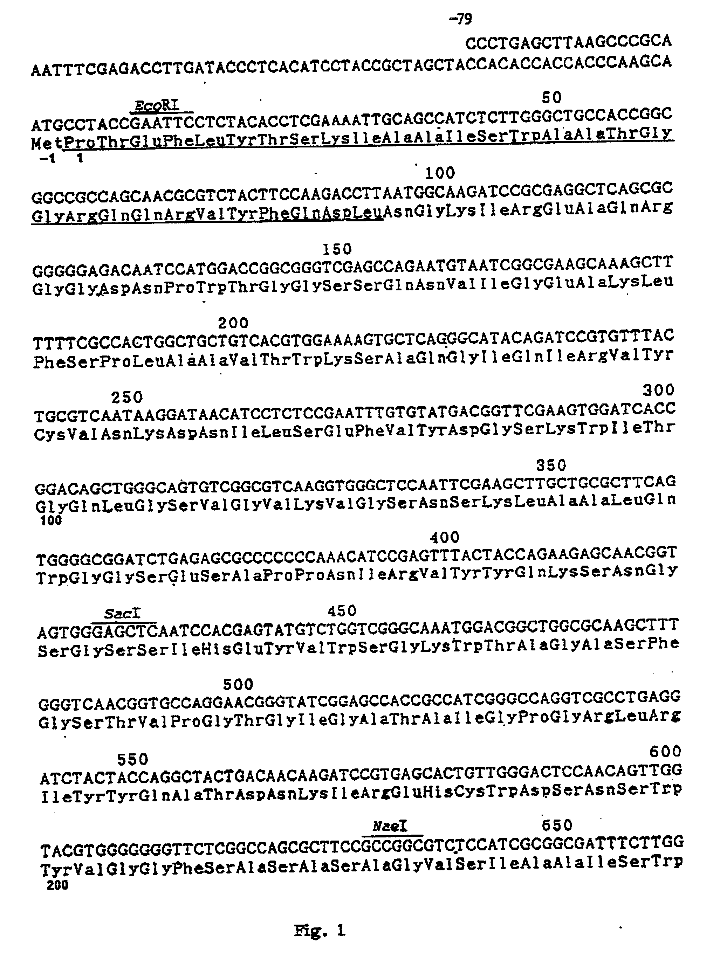

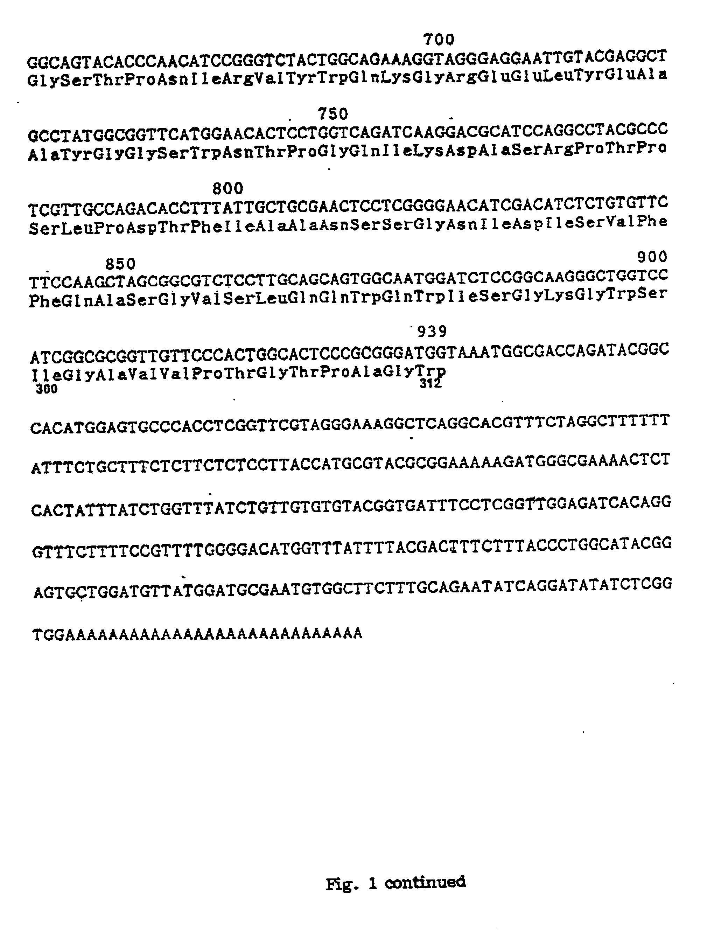

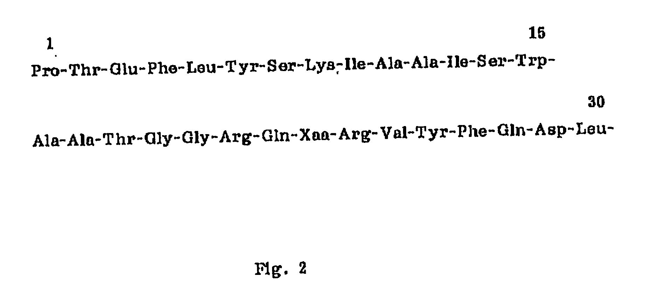

As described above, the cDNA of the present invention contains a region encoding AAL. The nucleotide sequence and the deduced amino acid sequence of the cDNA of an example of the cDNA of the present invention are shown in Fig. I. The cDNA shown in Fig. 1 contains a single open reading frame of 939 bases encoding a polypeptide of 313 amino acids. The ATG codon for methionine at nucleotides 80 - 82 represents the translation starting site. The amino acid sequence of the amino-terminal region of the natural AAL is shown in Fig. 2, and it completely matches with that deduced from the nucleotide sequence from the proline residue at position I (the underscored sequence in Fig. 1). The mature polypeptide consists of 312 amino acids, and its molecular weight is calculated to be 33,398, which is comparable to that of the AAL subunit determined by SDS-PAGE. The coding sequence of the cDNA insert is followed by 3′ non-coding region of 322 nucleotides with addition of a poly(A) tail.

It is well-recognized by those skilled in the art that a physiological activity of a polypeptide may not be substantially influenced even if a small number of amino acids of the polypeptide are substituted with other amino acids, if a small number of amino acids are deleted from the polypeptide, or if a small number of amino acids are added to the polypeptide. Therefore, even if a small number of bases of the above-described cDNA are substituted with other bases or deleted, or small number of bases are added to the base sequence so as to substitute a small number of amino acids of the polypeptide encoded by the DNA, to delete a small number of amino acids from the polypeptide or to add a small number of amino acids to the polypeptide, if the encoded polypeptide retains the specific binding ability to fucose and the ability of hemagglutination, the DNA is considered as an equivalent of the cDNA of the present invention and is included in the scope of the present invention. Further, as long as the region encoding the AAL is contained, the cDNA may contain one or more additional regions.

The recombinant vector of the present invention contains the above-described cDNA and enables the host cells to produce AAL encoded by the cDNA. The recombinant vector of the present invention contains, as the conventional expression vectors, a replication origin which enables the vector to replicate in the host cell, a promoter region necessary for the transcription, and in cases where the host cell is a prokaryotic cell, an SD sequence necessary for the translation, in addition to the above-described cDNA region. The recombinant vector preferably contains a selection marker such as a region giving drug resistance or temperature sensitivity, as well as a terminator. Vectors containing the replication origin, promoter, SD sequence, selection marker and the terminator are well-known in the art and many of them are commercially available. In the present invention, such commercially available vectors may be employed.

The cDNA and the recombinant vector of the present invention may be prepared as hereinbelow described, although the preparation method thereof is not restricted thereto.

First, all RNAs are extracted from the fruit bodies of Aleuria aurantia and mRNAs are recovered therefrom. This can be carried out by disrupting the fruit bodies of Aleuria aurantia in a solution containing a denaturing agent such as guanidium thiocyanate, recovering the RNA fractions, and passing the obtained RNA fractions through an oligo(dT) column. The thus obtained mRNAs are then treated with a reverse transcriptase in accordance with, for example, the method by Gubler and Hoffman [U. Gubler and B.J. Hoffman, Gene, 25, 263 (1983)] to synthesize the single cDNAs complementary to the respective mRNAs, and then double-stranded DNAs are formed using the cDNAs as templates. The resulting double-stranded DNAs are then provided with an appropriate restriction site such as EcoRI site by, for example, binding an appropriate linker, and the resultants are then respectively inserted in an appropriate vector such as λ gtII phage [R.A. Young and R.W. Davis, Proc. Natl. Acad, Sci. USA, 80, II94 (1984)]. The obtained recombinant vectors are then introduced into the host of the vectors (for example, in the case of λ gtII phage, E. coli - (e.g., E. coli YI090). The host cells are cultured and the cells in which the recombinant vector was introduced are selected. From the thus selected cells, the cells producing AAL are selected by using an antibody against AAL or a DNA fragment which is complementary to the mRNA encoding the nucleotide sequence corresponding to the amino acid sequence of AAL. In cases where a phage vector such as λ gt11 phage vector is employed, the desired DNA fragments may be obtained by selecting the phage which is expected to produce AAL in a plaque formed in an agar medium. Alternatively, without using a phage vector, a plasmid in which the cDNA of the present invention is inserted is prepared in accordance with the method by Okayama and Berg [H. Okayama and P. Berg, Mol. Cell Biol. 2, I6I (1982)], and the recombinant plasmid is introduced to a host such as E. coli.

The region encoding the AAL in the thus obtained recombinant vector may then be recombined with a vector which replicates and expresses the inserted region with high efficiency in a host cell.

AAL may be produced in large amount by transforming the host cells with the thus obtained recombinant vector and by culturing the transformants. The produced AAL may be purified by the precipitation with ammonium sulfate and by subsequent fucose-starch gel column chromatography [I. Matumoto and T. Osawa, Biochem. Biophys. Res. Commun. 46, I8I0 (1972)].

The invention will now be described by way of an example thereof. It should be understood that the example is presented for the illustration purpose only and should not be interpreted in any restrictive way.

In the example herein below described, unless otherwise specified, each operation was conducted in accordance with T. Maniatis et al., "Molecular Cloning. A Laboratory Manual" (1982), Cold Spring Harbor.

## [EXAMPLE]

### (I) Separation of mRNAs from Aleuria aurantia and Preparation of cDNAs

Ten grams of the fruit bodies of Aleuria aurantia collected locally and stored at -80°C were crushed with dry-ice, suspended in 32 ml of 5.5 M guanidine thiocyanate (GTC) solution and homogenized with a Teflon homogenizer. After centrifugation at 1000 x g for I0 minutes, 8 ml aliquot of the supernatant was overlaid on 3 ml of cesium-trifluoroacetate containing 0.1 M EDTA (density 1.50 g/ml) and centrifuged at 35,000 rpm for I6 hours at 15°C in a rotor (Hitachi RPS-40T rotor). The RNA pellet was dissolved in 4 M GTC solution and insoluble materials were removed by centrifugation. The RNA was precipitated with ethanol and dissolved in 10 mM Tris-1 mM EDTA (pH 8.0).

Poly (A) RNAs were collected by oligo(dT) cellulose chromatography and approximately 20 μg of poly-(A) RNA was converted to cDNA using a cDNA synthesis kit (Pharmacia LKB Biotechnology).

### (II) Preparation of Recombinant Phage Library and Screening Thereof

Resultant cDNAs were ligated with λ gtII and packaged in vitro. A cDNA library was constructed using E. coli strain YI090 [T.V. Huynh et al., "DNA cloning, vol. I, pp. I09-I35, IRL Press (1985), commercially available from Amersham] as a host and the library was screened immunologically with an anit-AAL serum against purified natural AAL. Immunopositive plaques were detected chromogenically using an immunoscreening system (commercially available from Amersham) and positive plaques were plaque-purified. That is, the host-cells were mixed with soft agar to form a plate and the proteins produced by the phages were tranferred to a nitrocellulose paper. Immunostaining was performed on the nitrocellulose paper using rabbit anti-AAL serum and anti-rabbit immunoglobulin to which peroxidase had been bound, to select one clone producing a protein which reacts with anit-AAL antibody. The clone was named λAAL-3.

### (III) Determination of Nucleotide Sequence of cDNA Encoding AAL

From the phage clone λ AAL-3 obtained in (II), a DNA fragment of about 1.3 kb which was expected to be originated from Aleuria aurantia was separated by digesting with EcoRI. The fragment was cloned in a plasmid vector for E. coli, pUCI8, and the nucleotide sequence thereof was determined by M-13 dideoxy method (the method by Sanger et al), which is shown in Fig. I.

On the other hand, according to the method by Kochibe and Furukawa (supra), AAL was extracted from the fruit bodies of Aleuria aurantia and the amino acid sequence thereof from the N-terminal to the 30th amino acid was determined using a commercially available peptide sequencer. The determined amino acid sequence is shown in Fig. 2. By comparing the amino acid sequence shown in Fig. I encoded by the cDNA and the amino acid sequence of the natural AAL shown in Fig. 2, it can be seen that these completely matches, so that it was proved that the above-described DNA fraction of about 1.3 kb encoded AAL.

### (IV) Preparation of Plasmid Producing AAL

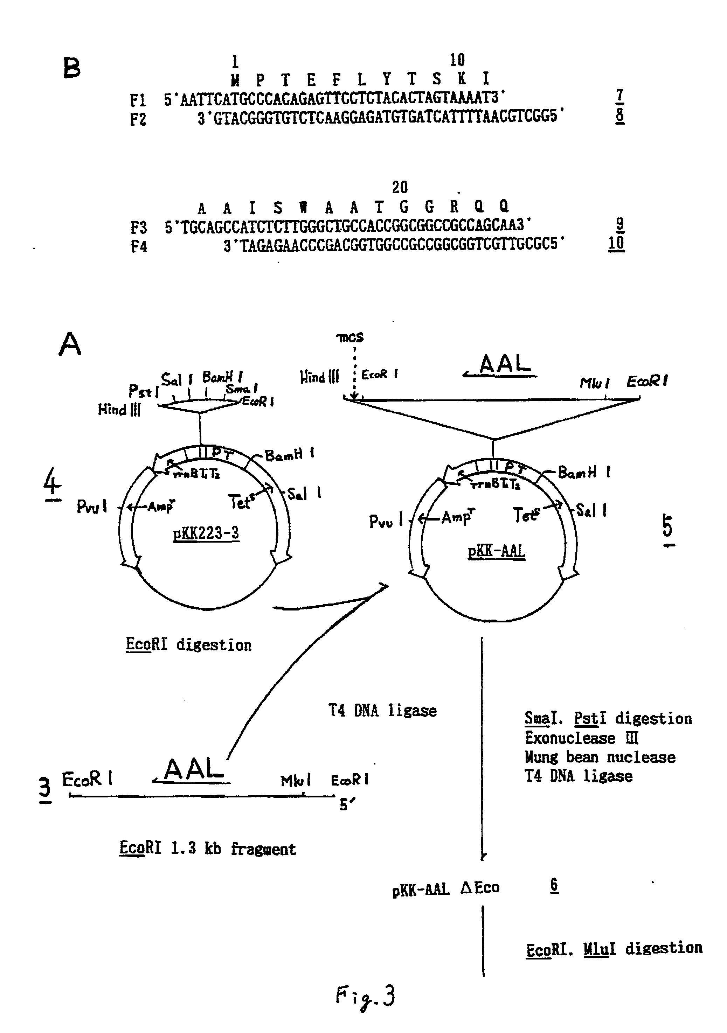

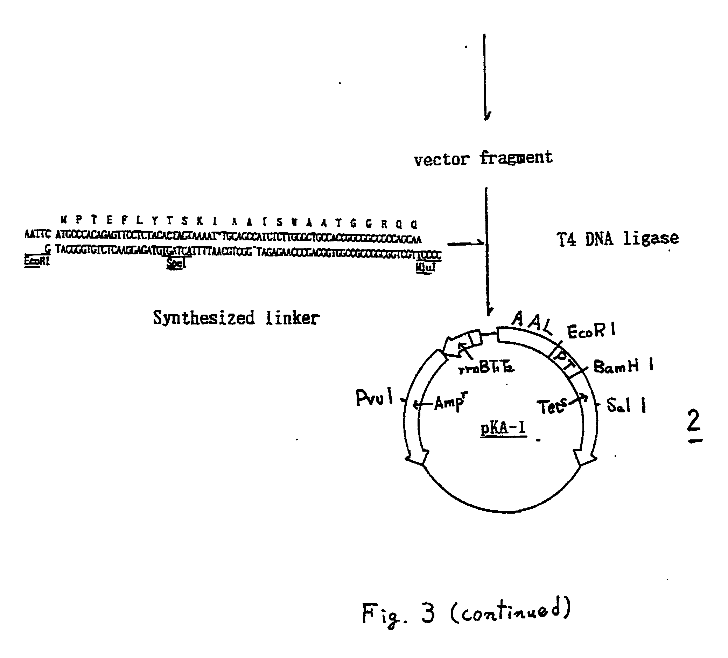

Referring to Figs. 3A and 3B, an AAL expression vector (2) was constructed using a I.3 kb EcoRI fragment (3) within the cDNA, chemically synthesized polynucleotides and plasmid pKK223-3 (4). A I.3 kb EcoRI fragment (position 89 - 1370) of the λ AAL-3 insert was inserted into plasmid pKK223-3. A plasmid (pKK-AAL (5)) in which the cDNA was inserted in the correct orientation was digested with Smal and PstI,

and treated with Exonuclease III followed by the treatment with Mung bean nuclease. After the treatment with Klenow fragment of DNA polymerase I, the polynucleotide was ligated by T4 DNA ligase. The resulting plasmid (pKK-AAL Δ Eco (6)) was cleaved with EcoRI and MluI, and the large fragment was isolated. Four nucleotides (7) - (10) shown in Fig. 3B were synthesized chemically, the sequence of which corresponded to the N-terminal part of AAL with the addition of one methionine at the amino-proximal end, EcoRI site at 5′-proximal end and MluI site at the distal end. The four fragments were annealed in the presence of isolated vector fragment described above and ligated to yield a new plasmid, pKA-1 (2).

(V) Production of AAL

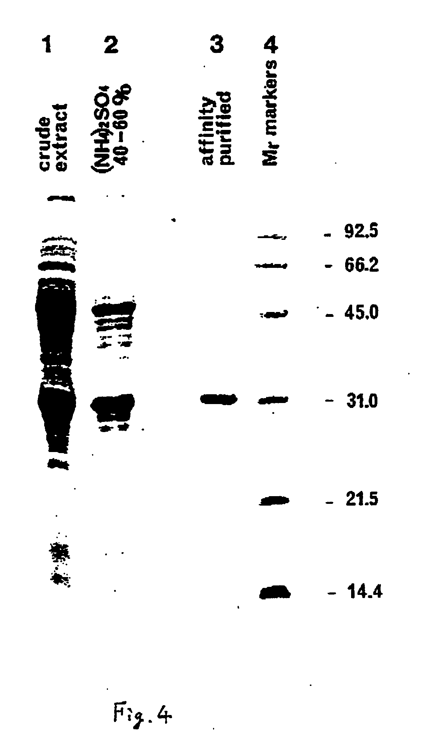

Referring to Table I and Fig. 4, AAL was obtained from transformed E. coli cells. That is, 5 liters of LB medium (Tryptone I0 g, Yeast extract 5 g and NaCl 5 g/liter, pH7.4) containing 100 μg/ml of ampicillin was inoculated with 50 ml of an overnight culture of E. coli JMI09 carrying the AAL expression vector pKA-1 described above. After the cell density reached to an A660 of I.0, isopropyl-$\beta$-D-galactoside was added to a final concentration of I mM, and the cells were grown for additional 16 hours at 30°C. Purification steps were all performed at 4°C. Harvested cells were suspended in 300 ml of phosphate-buffered saline (PBS: 8 mM $Na_2HPO_4$, 1.5 mM $KH_2PO_4$, pH 7.2, I37 mM NaCl, 2.7 mM KCl) and 100 ml aliquot of the suspension was sonicated three times for 10 minutes each. The cell lysate was centrifuged to remove the insoluble materials at 8000 x g for 20 minutes. To the supernatant (300 ml) was added ammonium sulfate and the fraction precipitated between 40% and 60% saturation was collected. The precipitate was dissolved in 80 ml of PBS and dialyzed against 2 liters of PBS for 24 hours. It was then mounted on a column (2.6 x 10 cm) of fucose-starch, prepared as described by Matumoto and Osawa (supra), which had been equilibrated with PBS and washed with 1 liter of PBS to remove unadsorbed materials. The lectin bound to the column was eluted with PBS containing 50 mM fucose. All the sugars described in this specification have D configuration except for fucose which has an L configuration. Active fractions were pooled and ammonium sulfate was added to 70% saturation. The precipitate was dissolved in 25 ml of PBS, dialyzed twice against 2 liters of PBS for 24 hours and finally adjusted to the volume of 30 ml with PBS.

TABLE 1

| Purification of rAAL | | | | | | |
|---|---|---|---|---|---|---|
| Step | Total volume (ml) | Total H.U[a] (units x 10⁻⁴) | Total Protein (mg) | Specific Activity (units/mg protein) | Yield | Purification ( -fold) |
| 1. Crude extract | 300 | 192 | 3360 | 571 | - | - |
| 2. Ammonium sulfate | 100 | 128 | 2120 | 604 | 0.67 | 1.06 |
| 3. Fucose-starch | 30 | 96 | 324 | 2963 | 0.50 | 5.19 |

[a] Homagglutination units (H.U.) as defined under "Experimental Procedures".

(VI) Characterization of AAL

The thus obtained AAL was characterized by a number of criteria. The specific activity (hemagglutination units/mg protein) of the prepared AAL was approximately 3000 units and its activity was inhibited by fucose, where the hemagglutination and inhibition assay were performed as follows: The titration of lectin was conducted by serially diluting the sample with PBS, and then mixing with an equal volume (25 μl) of 2% human type O erythrocytes suspended in PBS. Hemagglutination unit of a solution (H.U./ml) was defined as the reciprocal of the minimum amount of the solution (ml) giving positive reaction after 1 hour at room temperature. In inhibition assays, AAL (4 unites) was incubated at room temperature for 1 hour with saccharides to be tested which had been serially diluted with PBS and then assayed for the residual hemagllutinating activity. The minimum concentration of sugars that inhibited completely the hemagglutination was determined. The minimum concentration of fucose to exhibit the inhibition was 0.39 mM. Glucose, N-acetylglucosamine, galactose, mannose, N-acetylgalactosamine, lactose and α-methyl-

5

mannopyranoside showed no inhibition even at a concentration of 100 mM. *N*-Acetylneuraminic acid at a concentration of 25 mM did not show inhibition.

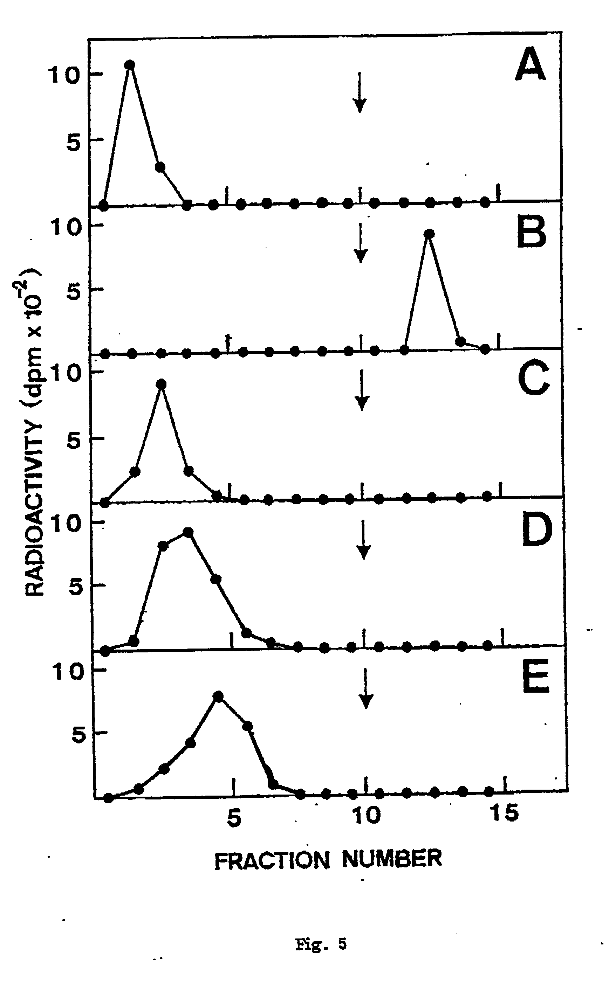

Sugar binding specificity of the AAL obtained as above was also investigated for oligosaccharide using a AAL-Sepharose column (the AAL in the column is obtained by the above-described process). Oligosaccharides used are listed in Table 2. Subscript OT is used to indicate $NaB_3H_4$-reduced oligosaccharide. All the oligosaccharides having $\alpha$-fucosyl residue linked at C-6 position of the proximal N-acetylglucosamine moiety (II, IV, V, VI, VIII and X) were retained in the column and eluted only with the buffer containing 0.5 mM fucose (Fig. 5B). The oligosaccharide lacking the fucosyl residue at the position (I, III, VII and IX) passed through the column without interaction (Fig. 5A). When three human milk oligosaccharides, i.e., LNF-I, LNF-II and LNF-III in Table 2, were subjected to the column, they were eluted as shown in Fig. 5C, D and E, respectively. These results strongly suggest that the AAL prepared as above has the same biological characteristics, and has the same binding characteristics toward fucosylated oligosaccharides as natural AAL.

Table 2  Structures of oligosaccharides, the behavior of which on an rAAL-Sepharose column was investigated.

$$R_1 = GlcNAc\beta1 \rightarrow 4GlcNAc_{OT} \qquad R_2 = \begin{array}{c} Fuc\alpha1 \\ \searrow 6 \\ GlcNAc\beta1 \rightarrow 4GlcNAc_{OT} \end{array}$$

| Structures | Abbreviated names |
|---|---|
| $Gal\beta1 \rightarrow 4GlcNAc\beta1 \rightarrow 2Man\alpha1 \searrow_6$ $Man\beta1 \rightarrow 4 \begin{cases} R_1 \\ R_2 \end{cases}$ $Gal\beta1 \rightarrow 4GlcNAc\beta1 \rightarrow 2Man\alpha1 \nearrow^3$ | I, II |
| $Gal\beta1 \rightarrow 4GlcNAc\beta1 \rightarrow 2Man\alpha1 \searrow_6$ $GlcNAc\beta1 \rightarrow 4Man\beta1 \rightarrow 4 \begin{cases} R_1 \\ R_2 \end{cases}$ $Gal\beta1 \rightarrow 4GlcNAc\beta1 \rightarrow 2Man\alpha1 \nearrow^3$ | III, IV |
| $Man\alpha1 \searrow_6$ $Man\beta1 \rightarrow 4R_2$ $Gal\beta1 \rightarrow 4GlcNAc\beta1 \rightarrow 2Man\alpha1 \nearrow^3$ | V |
| $Gal\beta1 \rightarrow 4GlcNAc\beta1 \rightarrow 2Man\alpha1 \searrow_6$ $Man\beta1 \rightarrow 4R_2$ $Man\alpha1 \nearrow^3$ | VI |
| $Gal\beta1 \rightarrow 4GlcNAc\beta1 \searrow_6$ $Man\alpha1 \searrow_6$ $Gal\beta1 \rightarrow 4GlcNAc\beta1 \nearrow^2$ $Man\beta1 \rightarrow 4 \begin{cases} R_1 \\ R_2 \end{cases}$ $Gal\beta1 \rightarrow 4GlcNAc\beta1 \rightarrow 2Man\alpha1 \nearrow^3$ | VII, VIII |
| $Gal\beta1 \rightarrow 4GlcNAc\beta1 \searrow_6$ $Man\alpha1 \searrow_6$ $Gal\beta1 \rightarrow 4GlcNAc\beta1 \nearrow^2$ $Man\beta1 \rightarrow 4 \begin{cases} R_1 \\ R_2 \end{cases}$ $Gal\beta1 \rightarrow 4GlcNAc\beta1 \searrow_4$ $Man\alpha1 \nearrow^3$ $Gal\beta1 \rightarrow 4GlcNAc\beta1 \nearrow^2$ | IX, X |
| $Fuc\alpha1 \rightarrow 2Gal\beta1 \rightarrow 3GlcNAc\beta1 \rightarrow 3Gal\beta1 \rightarrow 4Glc_{OT}$ | LNF I |
| $\begin{array}{c} Gal\beta1 \rightarrow 3GlcNAc\beta1 \rightarrow 3Gal\beta1 \rightarrow 4Glc_{OT} \\ 4 \\ \uparrow \\ Fuc\alpha1 \end{array}$ | LNF II |
| $\begin{array}{c} Gal\beta1 \rightarrow 4GlcNAc\beta1 \rightarrow 3Gal\beta1 \rightarrow 4Glc_{OT} \\ 3 \\ \uparrow \\ Fuc\alpha1 \end{array}$ | LNF III |

7

Although the present invention has been described with reference to the preferred embodiment, it should be understood that various modifications and variations can be easily made by those skilled in the art without departing from the spirit of the present invention.

## Claims

1. A cloned cDNA comprising a region encoding Aleuria aurantia lectin.

2. The cDNA of claim I, which encodes the polypeptide with an amino acid sequence shown in Fig. I.

3. The cDNA of claim I, which has a nucleotide sequence shown in Fig. I.

4. A recombinant vector containing the cDNA of any one of claims 1 - 3, which can express the cDNA in a host cell to produce Aleuria aurantia lectin.

5. The recombinant vector of claim 4, further comprising a codon encoding methionine at the N-terminal of the Aleuria aurantia lectin.

6. The recombinant vector of claim 5, which is pKA-1.

7. A process for preparing Aleuria aurantia lectin which comprises inserting the cDNA as defined in anyone of claims 1 to 3 into a suitable expression vector, transferring the expression vector into a suitable host, cultivating said host and isolating the expressed recombinant Aleuria aurantia lectin.

8. Aleuria aurantia lectin obtainable according to the process of claim 7.

9. The use of Aleuria aurantia lectin according to claim 8 for preparing a diagnostic composition or as a reagant for separating or analyzing sugar chains.

-79

CCCTGAGCTTAAGCCCGCA
AATTTCGAGACCTTGATACCCTCACATCCTACCGCTAGCTACCACACCACCACCCAAGCA

EcoRI                                    50
ATGCCTACCGAATTCCTCTACACCTCGAAAATTGCAGCCATCTCTTGGGCTGCCACCGGC
MetProThrGluPheLeuTyrThrSerLysIleAlaAlaIleSerTrpAlaAlaThrGly
-1  1

                                         100
GGCCGCCAGCAACGCGTCTACTTCCAAGACCTTAATGGCAAGATCCGCGAGGCTCAGCGC
GlyArgGlnGlnArgValTyrPheGlnAspLeuAsnGlyLysIleArgGluAlaGlnArg

                   150
GGGGGAGACAATCCATGGACCGGCGGGTCGAGCCAGAATGTAATCGGCGAAGCAAAGCTT
GlyGlyAspAsnProTrpThrGlyGlySerSerGlnAsnValIleGlyGluAlaLysLeu

              200
TTTTCGCCACTGGCTGCTGTCACGTGGAAAAGTGCTCAGGGCATACAGATCCGTGTTTAC
PheSerProLeuAlaAlaValThrTrpLysSerAlaGlnGlyIleGlnIleArgValTyr

     250                                          300
TGCGTCAATAAGGATAACATCCTCTCCGAATTTGTGTATGACGGTTCGAAGTGGATCACC
CysValAsnLysAspAsnIleLeuSerGluPheValTyrAspGlySerLysTrpIleThr

                                    350
GGACAGCTGGGCAGTGTCGGCGTCAAGGTGGGCTCCAATTCGAAGCTTGCTGCGCTTCAG
GlyGlnLeuGlySerValGlyValLysValGlySerAsnSerLysLeuAlaAlaLeuGln
100

                          400
TGGGGCGGATCTGAGAGCGCCCCCCCCAAACATCCGAGTTTACTACCAGAAGAGCAACGGT
TrpGlyGlySerGluSerAlaProProAsnIleArgValTyrTyrGlnLysSerAsnGly

     SacI              450
AGTGGGAGCTCAATCCACGAGTATGTCTGGTCGGGCAAATGGACGGCTGGCGCAAGCTTT
SerGlySerSerIleHisGluTyrValTrpSerGlyLysTrpThrAlaGlyAlaSerPhe

              500
GGGTCAACGGTGCCAGGAACGGGTATCGGAGCCACCGCCATCGGGCCAGGTCGCCTGAGG
GlySerThrValProGlyThrGlyIleGlyAlaThrAlaIleGlyProGlyArgLeuArg

     550                                          600
ATCTACTACCAGGCTACTGACAACAAGATCCGTGAGCACTGTTGGGACTCCAACAGTTGG
IleTyrTyrGlnAlaThrAspAsnLysIleArgGluHisCysTrpAspSerAsnSerTrp

                        NaeI          650
TACGTGGGGGGGGTTCTCGGCCAGCGCTTCCGCCGGCGTCTCCATCGCGGCGATTTCTTGG
TyrValGlyGlyPheSerAlaSerAlaSerAlaGlyValSerIleAlaAlaIleSerTrp
200

Fig. 1

700
GGCAGTACACCCAACATCCGGGTCTACTGGCAGAAAGGTAGGGAGGAATTGTACGAGGCT
GlySerThrProAsnIleArgValTyrTrpGlnLysGlyArgGluGluLeuTyrGluAla

750
GCCTATGGCGGTTCATGGAACACTCCTGGTCAGATCAAGGACGCATCCAGGCCTACGCCC
AlaTyrGlyGlySerTrpAsnThrProGlyGlnIleLysAspAlaSerArgProThrPro

800
TCGTTGCCAGACACCTTTATTGCTGCGAACTCCTCGGGGAACATCGACATCTCTGTGTTC
SerLeuProAspThrPheIleAlaAlaAsnSerSerGlyAsnIleAspIleSerValPhe

850                                                    900
TTCCAAGCTAGCGGCGTCTCCTTGCAGCAGTGGCAATGGATCTCCGGCAAGGGCTGGTCC
PheGlnAlaSerGlyValSerLeuGlnGlnTrpGlnTrpIleSerGlyLysGlyTrpSer

939
ATCGGCGCGGTTGTTCCCACTGGCACTCCCGCGGGATGGTAAATGGCGACCAGATACGGC
IleGlyAlaValValProThrGlyThrProAlaGlyTrp
300                                    312

CACATGGAGTGCCCACCTCGGTTCGTAGGGAAAGGCTCAGGCACGTTTCTAGGCTTTTTT

ATTTCTGCTTTCTCTTCTCTCCTTACCATGCGTACGCGGAAAAAGATGGGCGAAAACTCT

CACTATTTATCTGGTTTATCTGTTGTGTGTACGGTGATTTCCTCGGTTGGAGATCACAGG

GTTTCTTTTCCGTTTTGGGGACATGGTTTATTTTACGACTTTCTTTACCCTGGCATACGG

AGTGCTGGATGTTATGGATGCGAATGTGGCTTCTTTGCAGAATATCAGGATATATCTCGG

TGGAAAAAAAAAAAAAAAAAAAAAAAAAAAAAAAA

Fig. 1 continued

1

Pro-Thr-Glu-Phe-Leu-Tyr-Ser-Lys-Ile-Ala-Ala-Ile-Ser-Trp-

15

Ala-Ala-Thr-Gly-Gly-Arg-Gln-Xaa-Arg-Val-Tyr-Phe-Gln-Asp-Leu-

30

Fig. 2

EP 0 387 861 A2

**B**

```
            1                           10
            M  P  T  E  F  L  Y  T  S  K  I
F1   5´AATTCATGCCCACAGAGTTCCTCTACACTAGTAAAAT3´              7
                                                           ─
F2        3´GTACGGGTGTCTCAAGGAGATGTGATCATTTTAACGTCGG5´     8
```

```
                        20
       A  A  I  S  W  A  A  T  G  G  R  Q  Q
F3   5´TGCAGCCATCTCTTGGGCTGCCACCGGCGGCCGCCAGCAA3´          9
                                                          ──
F4       3´TAGAGAACCCGACGGTGGCCGCCGGCGGTCGTTGCGC5´        10
```

**A**

EcoRI digestion

T4 DNA ligase

EcoRI 1.3 kb fragment

SmaI. PstI digestion
Exonuclease III
Mung bean nuclease
T4 DNA ligase

pKK-AAL ΔEco        6

EcoRI. MluI digestion

Fig.3

vector fragment

T4 DNA ligase

M P T E F L Y T S K I A A I S W A A T G G R Q Q

AATTC ATGCCCACAGAGTTCCTCTACACTAGTAAAAT TGCAGCCATCTCTTGGCCTGCCACGGCGGCCCCCAGCAA
   G TACGGGTGTCTCAAGGAGATGTGATCATTTTAACGTCGG TAGAGAACCCGACGGTGGCCGCGGGGGTCGTTCCCC
EcoRI                                  Sec I                                           Mlu I

Synthesized linker

Fig. 3 (continued)

Fig. 4

Fig. 5